# EUROPÄISCHE PATENTSCHRIFT

(11) **EP 2 219 543 B1**
(45) Veröffentlichungstag und Bekanntmachung des Hinweises auf die Patenterteilung: **16.07.2014**
(21) Anmeldenummer: 08859629.1
(22) Anmeldetag: 11.12.2008
(51) Int. Cl.: A61B 18/12, A61B 17/3203, A61B 19/00

(54) **VORRICHTUNG ZUR KONTAKTLOSEN KOMMUNIKATION**
DEVICE FOR CONTACTLESS COMMUNICATION
DISPOSITIF POUR COMMUNIQUER SANS CONTACT

(30) Priorität: 12.12.2007 DE 102007059619; 10.12.2008 DE 102008061418
(43) Veröffentlichungstag der Anmeldung: 25.08.2010
(73) Patentinhaber: ERBE Elektromedizin GmbH, 72072 Tübingen (DE)
(72) Erfinder: BELLER, Jürgen, 72810 Gomaringen (DE); SCHNITZLER, Uwe, 72074 Tübingen (DE); SELIG, Peter, 72379 Hechingen (DE)
(74) Vertreter: Bohnenberger, Johannes
(86) Internationale Anmeldenummer: PCT/EP2008/010550
(87) Internationale Veröffentlichungsnummer: WO 2009/074329

(56) Entgegenhaltungen:
- EP-A- 1 410 766
- EP-A- 1 943 957
- EP-A- 1 958 586
- WO-A-00/24318
- WO-A-2006/031632
- WO-A-2006/060781
- DE-A1-102005 011 385
- DE-U1- 9 200 452
- US-A- 5 383 460
- US-A1- 2001 051 766
- US-A1- 2004 122 419
- US-A1- 2007 066 978
- US-A1- 2007 167 941

## Beschreibung

Die Erfindung betrifft eine Vorrichtung zur kontaktlosen Kommunikation zwischen einem Chirurgiegerät und mindestens einem mit diesem anwendbaren chirurgischen Instrument oder dergleichen Zubehöreinrichtung sowie die Verwendung einer beschreibbaren und auslesbaren, mit einer Instrumentenantenne in Verbindung stehenden Speichereinrichtung.

Im Bereich der Hochfrequenzchirurgie (HF-Chirurgie), Kryochirurgie oder auch Wasserstrahlchirurgie werden eine Vielzahl von Instrumenten eingesetzt, die innerhalb eines chirurgischen Systems mit einem Chirurgiegerät betreibbar sind. Derartige Instrumente sind z. B. Elektroden aufweisende Instrumente für die HF-Chirurgie oder auch Applikatoren für die Zufuhr von Wasser an das Operationsgebiet. Sonden werden insbesondere im Bereich der Endoskopie eingesetzt.

Aufgrund der Vielzahl verschiedenster Instrumente bzw. Zubehörteile eines chirurgischen Systems ist es erforderlich, eine automatische Erkennung der Instrumente vorzusehen, so dass das entsprechende Chirurgiegerät die für den Betrieb des jeweiligen Instruments erforderlichen Parameter, wie z. B. geeignete Spannung oder Wasserdruck zur Verfügung stellen kann. Andernfalls müsste der Anwender sämtliche Parameter selbst per Hand eingeben bzw. einstellen, d. h., es wäre vor der eigentlichen Benutzung eine zeitaufwändige Konfigurationsphase erforderlich. Zudem kann die manuelle Eingabe eine für den Patienten gefährliche Fehlerquelle darstellen, insbesondere deshalb, weil der Anwender nicht alle Werte parat haben wird.

Aus dem Stand der Technik sind bisher verschiedene Lösungen für eine automatische Instrumentenerkennung bekannt. So werden beispielsweise elektronische Speicher, wie ein EEPROM, am Instrument vorgesehen, um Daten über das Instrument an das Chirurgiegerät übermitteln zu können. Die Instrumentenerkennung wird mittlerweile per Kabelverbindung, aber auch drahtlos angewendet.

Das EEPROM benötigt mindestens einen zusätzlichen Steckkontakt, um die elektrische Verbindung zwischen Gerät und dem Speicherchip im Instrument bzw. im Zubehörteil herstellen zu können. Daher ist die Anwendung in einem Standard-Steckverbinder, wie in einem aus der HF-Chirurgie bekannten Neutralelektroden- oder 3-Pin-Stecker nicht möglich.

Aus der DE 10 2005 044 918 A1 ist eine Vorrichtung zur kontaktlosen Identifikation und Kommunikation zwischen einem HF-Generator und einem an diesen angeschlossenen Instrument bekannt. Über eine Transpondereinrichtung (mit Instrumentenantenne), die in einem Instrumentenstecker angeordnet ist und eine Schreib- und Leseeinheit (mit Generatorantenne), die wiederum am Generator angeordnet ist, lassen sich nun Informationen per Funkkontakt zwischen Instrument und HF-Generator austauschen. Der einschreibbare und auslesbare Datenspeicher der Transpondereinrichtung ist hier jedoch nur für einen beschränkten Informationsaustausch geeignet.

Aus der WO 2006/03162 A ist eine medizinische Laserbehandlungseinrichtung bekannt, die sich in zwei Komponenten aufteilen lässt, nämlich eine Versorgungseinrichtung und ein Applikationsgerät. Das Applikationsgerät besteht aus einem Handgriff und einer an diesem abnehmbar befestigten Spitze, die je nach Bedarf und Anwendung ausgetauscht werden kann. Die Spitze hat eine Speichereinrichtung, die das Auslesen von relevanten Parametern bezüglich der jeweils aufgesetzten Spitze ermöglicht, wobei die Speichereinrichtung vorzugsweise dafür verwendet wird, Konfigurationsdaten, die sich auf die Spitze beziehen, zu speichern. Eine Nutzung dieser Speichereinrichtung zur Erfassung von aktuellen Parametern geht aus dieser Druckschrift nicht hervor.

Der Erfindung liegt die Aufgabe zu Grunde, eine Vorrichtung der eingangs genannten Art dahin gehend aufzuzeigen, dass der Datenaustausch verbessert, insbesondere erweitert und so die Sicherheit des Patienten gesteigert wird.

Diese Aufgabe wird durch eine Vorrichtung zur kontaktlosen Kommunikation zwischen einem Chirurgiegerät und mindestens einem mit diesem anwendbaren chirurgischen Instrument nach Patentanspruch 1 gelöst. Insbesondere wird die Aufgabe vorrichtungsmäßig durch eine Vorrichtung zur kontaktlosen Kommunikation zwischen einem Chirurgiegerät und mindestens einem mit diesem anwendbaren chirurgischen Instrument oder dergleichen Zubehöreinrichtung gelöst, wobei das Chirurgiegerät eine Steuerungs- und Auswerteeinrichtung aufweist, der mindestens eine Schreib- und/oder Leseeinrichtung zugeordnet ist, die mit einer Geräteantenne in Verbindung steht, wobei dem Instrument mindestens eine, insbesondere als

RFID-Transponder ausgebildete, beschreibbare und auslesbare Speichereinrichtung zugeordnet ist (bzw. der Transponder umfasst die Speichereinrichtung), die mit einer Instrumentenantenne in Verbindung steht, so dass Daten zwischen der Schreib- und/oder Leseeinrichtung des Chirurgiegeräts und der Speichereinrichtung des Instruments über eine drahtlose Kommunikation austauschbar sind, wobei das Instrument eine Mess- und/oder Aktuatoreinrichtung umfasst, die der Speichereinrichtung zugeordnet ist und die derart die Speichereinrichtung beeinflussend ausgebildet ist, dass mindestens ein Parameter am und/oder im Instrument und/oder an einem zu behandelnden Gewebe zur Übermittlung an das Chirurgiegerät erfassbar ist und/oder wobei das mindestens eine Signal bzw. ganz allgemein Daten zur Beeinflussung der Instrumentenfunktion vom Chirurgiegerät an das Instrument über das RFID-System übermittelbar ist bzw. sind.

Ein wesentlicher Punkt der Erfindung liegt darin, dass durch das Zusammenwirken von Transponder (auch "Tag" genannt) und Mess- und/oder Aktuatoreinrichtung nun nicht nur elektronisch gespeicherte Informationen zwischen Instrument und Chirurgiegerät bzw. zwischen Schreib- und/oder Leseeinrichtung und beschreibbarer und auslesbarer Speichereinrichtung ausgetauscht, sondern auch Messdaten und digitale Signale ausgewertet und übertragen werden können. Dies ist insofern möglich, als das Transponder-Bauteil außer einem beschreibbaren und auslesbaren Speicherbereich zusätzlich digitale und analoge Schnittstellen aufweist, mit denen Sensoren, Aktoren und allgemein elektronische Baugruppen am Instrument oder im Instrumentenkabel betrieben und ausgewertet werden können (z. B. Tag mit integrierter Messtechnik). Die Tags weisen hierzu Schnittstellen auf, über die die Messdaten erfasst werden können. Das heißt, dass weitere wesentliche Informationen auf einfachste Weise an das Chirurgiegerät übertragbar sind, ohne hierfür zusätzliche Leitungen vorsehen zu müssen. Daten werden natürlich auch in umgekehrter Richtung übertragen, also vom Chirurgiegerät zum Instrument. Die Messeinrichtung erlaubt die Erfassung eines umfassenden Datenspektrums und daher auch eine umfassende Patientenbetreuung. Zudem kann aufgrund der Anwendung der RFID-Technologie auch bei chirurgischen Systemen, die nicht mit hochfrequentem Strom arbeiten, eine Datenübermittlung stattfinden, auch wenn keine elektrischen Kontakte für die Instrumente bzw. die Zubehöreinrichtungen vorgesehen sind. So verfügen Instrumente bzw. Zubehöreinrichtungen für die Kryochirurgie oder die Wasserstrahlchirurgie nur über einen pneumatischen oder fluidischen Anschluss. Ein Datentransfer ist mit der erfindungsgemäßen Vorrichtung trotzdem möglich.

Konkret heißt dies nun, dass die Mess- und/oder Aktuatoreinrichtung derart der Speichereinrichtung zugeordnet ist, dass die von ihr erfassten Daten (z. B. Messwerte oder auch detektierte Aktionen) dem Chirurgiegerät bzw. der Schreib- und/oder Leseeinrichtung übermittelt werden können, so dass daraufhin - sofern notwendig - Einstellungen am Chirurgiegerät bzw. am Generator vornehmbar sind (selbsttätig oder ggf. auch manuell) und das Instrument daraufhin über das Instrumentenkabel z. B. mit einer anderen geeigneten Spannung bzw. einem anderen Strom versorgt wird. Zudem (mit der Übermittlung entsprechender Parameter über das Instrumentenkabel oder auch ohne diese Übermittlung) lassen sich Daten über das RFID-System vom Chirurgiegerät bzw. von der Schreib- und/oder Leseeinrichtung an das Instrument übermitteln, so dass beispielsweise die Aktuatoreinrichtung über den Tag betätigbar ist oder betätigt wird. Die von der Schreib- und/oder Leseeinrichtung übermittelten Daten können aber auch Reaktionen am Instrument bewirken, ohne dass die Mess- und/oder Aktuatoreinrichtung aktiv wird. In jedem Falle ist der Datentransfer in beide Richtungen möglich, wobei über das Instrumentenkabel gleichzeitig mit dem Datentransfer oder ausschließlich (also ohne Datentransfer von der Schreib- und/oder Leseeinrichtung an die Speichereinrichtung) entsprechende Parameter am Instrument bereitgestellt werden können.

Die RFID-Technologie mit der kombinierten Mess- und/oder Aktuatoreinrichtung bietet sich insbesondere auch für Instrumente an, die über einen standardisierten Steckverbinder mit dem Chirurgiegerät verbindbar sind. Diese Steckverbinder weisen nicht genügend Kontakte zur Übermittlung zusätzlicher Daten auf. Die zusätzlichen Daten (also z. B. von der Messeinrichtung) können nun über die drahtlose Verbindung des RFID-Systems übertragen werden. Die Stromversorgung der Elektronik auf der Instrumentenseite kann in einem bestimmten Rahmen ebenfalls durch dieses erfolgen.
Die RFID-Technologie bietet die Möglichkeit, einen nicht-flüchtigen elektronischen Speicher (EEPROM) drahtlos über eine Funkverbindung zu beschreiben und auszulesen. Der Vorteil dabei ist, dass der Speicher nicht unbedingt eine Spannungsversorgung in Form einer Batterie benötigt, da er die notwendige elektrische Energie aus dem Trägersignal der Funkverbindung beziehen kann.

Es sei darauf hingewiesen, dass der Datenaustausch zwischen der Schreib- und/oder Leseeinrichtung und der Speichereinrichtung über die jeweiligen Antennen erfolgt, auch wenn ganz allgemein von einem Datenaustausch zwischen Chirurgiegerät und Instrument die Rede ist.

Erfindungsgemäß ist die beschreibbare und auslesbare Speichereinrichtung mit der Instrumentenantenne als ein Transponder-IC mit einem frei programmierbaren Mikroprozessor ausgebildet ist. Das heißt, es können bei dieser Art des Transponders jederzeit neue Daten eingegeben, überschrieben oder zusätzlich, neben den bestehenden eingegeben werden. So auch Betriebsdaten, die eine weitere Benutzung des Instruments bzw. des Zubehörteils erleichtern.

Auch ein fest programmierter Transponder-Speicher wäre denkbar. Dies kann z. B. dann vorteilhaft sein, wenn das Ändern der Daten im Speicher, beispielsweise durch den Anwender, verhindert werden soll.

Vorzugsweise ist die beschreibbare und auslesbare Speichereinrichtung mit der Instrumentenantenne in einer Steckereinrichtung oder in einem Verbindungskabel zum Verbinden des Instruments mit dem Chirurgiegerät ausgebildet. Sobald nun das Instrument mit dem Chirurgiegerät verbunden wird, sind Geräteantenne und Instrumentenantenne in einem geeigneten Abstand und in geeigneter Ausrichtung zueinander angeordnet, so dass eine optimale Kommunikation zwischen Schreib- und/oder Leseeinrichtung und Speichereinrichtung gegeben ist.

Alternativ wäre es natürlich möglich, den Transponder mit der Instrumentenantenne im Instrument anzuordnen. Hier könnte die Kommunikation allerdings aufgrund unterschiedlicher Positionen des Instruments, z. B. während eines chirurgischen Eingriffs, erschwert werden.

Beim Herstellungsprozess des Steckers kann der Tag einfach in den im Spritzgussverfahren hergestellten Stecker eingegossen werden, d. h., der aufwändige Konfektionierungs- und Kontaktierungsprozess des EEPROM's entfällt, wodurch die Herstellkosten auf Instrumentenseite reduziert werden.

Erfindungsgemäß ist der Mess- einrichtung mindestens ein instrumentenseitig angeordneter Sensor zugeordnet und die Mess- einrichtung ist derart ausgebildet, dass sie ein Sensorsignal mindestens eines Sensors als den Parameter erfasst. Somit können auf einfache Weise, nämlich über einen geeignet platzierten Sensor, Daten erfasst und ausgewertet werden.

Weiterhin kann der Mess- und/oder Aktuatoreinrichtung mindestens ein instrumentenseitig angeordneter Aktuator zur Beeinflussung der Instrumentfunktion zugeordnet sein. Ein derartiger Aktuator ist z. B. ein Schalter oder ein Schieber in einem Ventil, um so den Betrieb des Instruments zu regeln bzw. zu steuern. Vorzugsweise wird die Speichereinrichtung des Instruments den Befehl vom Chirurgiegerät aufnehmen und über eine entsprechende Schnittstelle an den Aktuator weiterleiten. Der Aktuator wird so also über den Transponder betätigt.

Sobald die Mess- und/oder Aktuatoreinrichtung im Rahmen ihrer Messfunktion ein Sensorsignal erfasst hat, kann dieses in dem Tag gespeichert und über drahtlose Kommunikation an die Schreib- und/oder Leseeinrichtung übermittelt werden. Die Steuerungs- und Auswerteeinrichtung im Chirurgiegerät wertet das empfangene Signal aus und kann daraufhin eine entsprechende Einstellung (sofern notwendig) am Chirurgiegerät vornehmen. Auch kann beispielsweise ein Funksignal zur Betätigung des Aktuators über das RFID-System dienen, um so das Instrument funkgesteuert zu bedienen, wie bereits oben dargelegt.

Vorzugsweise ist die Mess- und/oder Aktuatoreinrichtung bzw. die Vorrichtung zur kontaktlosen Kommunikation derart ausgebildet, dass die Erfassung des Parameters und die Beeinflussung der Instrumentenfunktion unabhängig voneinander ausführbar sind. Das heißt, sowohl die Messeinrichtung, als auch die Aktuatoreinrichtung können unabhängig voneinander betrieben werden. Die etwaige Erfassung eines Sensorsignals kann also Einstellungen am Chirurgiegerät bewirken, ohne dass der mindestens eine Aktuator betätigt wird (es kann z. B. ein Parameter durch die Messeinrichtung erfasst und an das Chirurgiegerät übermittelt werden, ohne dass ein Aktuator tätig ist oder wird).

Möglich ist es natürlich auch, dass die Mess- und/oder Aktuatoreinrichtung bzw. die Vorrichtung zur kontaktlosen Kommunikation derart ausgebildet ist, dass die Beeinflussung der Instrumentenfunktion aufgrund eines erfassten Parameters erfolgt. Das kann also bedeuten, dass die Messeinrichtung der Mess- und/oder Aktuatoreinrichtung z. B. über einen Sensor ein Signal erfasst. Das Signal wird dann an die Schreib- und/oder Leseeinrichtung übermittelt, so dass über diese aufgrund des erfassten Signals eine Ansteuerung des Aktuators über das RFID-System möglich ist. Auch kann der Aktuator "per Draht" über eine Signalleitung angesteuert werden. Die Betätigung der Messeinrichtung aufgrund einer Aktion des Aktuators ist ebenfalls möglich, d. h., verschiedenste Varianten können hier vorgesehen werden.

In einer weiteren Ausführungsform ist die Mess- und/oder Aktuatoreinrichtung im Instrument oder in dem Verbindungskabel zum Verbinden des Instruments mit dem Chirurgiegerät ausgebildet. Dies erleichtert u. a. die Zuordnung der instrumentenseitig angeordneten Sensoren und/oder Aktoren.

Auch kann die Mess- und/oder Aktuatoreinrichtung derart ausgebildet sein, dass sie die Betätigung eines Schalters (z. B. durch den Chirurgen) am Instrument als den Parameter erfasst. So ist es also möglich, dass der Chirurg mit Betätigung des Schalters einen Vorgang auslösen möchte (z. B. das Wechseln von einem Koagulationsvorgang zu einem Schneidvorgang), und diese Information nun über die drahtlose Kommunikation an das Chirurgiegerät übermittelt werden kann. Am Chirurgiegerät werden dann die neuen Einstellungen (z. B. Erhöhung der Spannung) selbsttätig vorgenommen und z. B. über die elektrische Verbindung am Instrument zur Verfügung gestellt.

Eine Ausführungsform sieht z. B. vor, dass das Chirurgiegerät und das Instrument für die Wasserstrahlchirurgie ausgebildet sind und die Mess- und/oder Aktuatoreinrichtung derart ausgebildet und angeordnet ist, dass der Druck in einer Zuleitung für Wasser an das zu behandelnde Gewebe eines Wasserstrahlapplikators als Parameter, vorzugsweise über den Sensor, erfassbar ist. Ein Sensor am Instrument könnte also Informationen über den anliegenden Druck liefern, der z. B. kontinuierlich gemessen wird und diese Informationen über die drahtlose Kommunikation an das Chirurgiegerät bzw. dessen Schreib- und/oder Leseeinrichtung weitergeben. Daraufhin kann der Druck selbsttätig, ggf. auch manuell (z. B. aufgrund einer Anzeige am Chirurgiegerät) geregelt werden.

Die Steuerungs- und Auswerteeinrichtung, die dem Chirurgiegerät zugeordnet ist, kann die von der Schreib- und/oder Leseeinrichtung erfassten Daten auswerten und das Chirurgiegerät entsprechend ansteuern, um erforderliche Steuer- oder Regelvorgänge auszulösen. Damit ist z. B. gewährleistet, dass stets der richtige Wasserdruck am Operationsgebiet vorliegt.

Vorzugsweise können das Chirurgiegerät und das Instrument für die HF-Chirurgie ausgebildet sein, wobei die Mess- und/oder Aktuatoreinrichtung dann derart ausgebildet und angeordnet ist, dass die Temperatur einer Elektrode eines HF-chirurgischen Instruments als Parameter (oder auch die Temperatur des behandelten Gewebes) während einer Benutzungshandlung, vorzugsweise über den Sensor, erfassbar ist. Über die Temperaturerfassung kann so eine geeignete Spannung für etwaige Schneid- oder Koagulationsvorgänge bereitgestellt werden.

Eine weitere Ausführungsform sieht vor, dass die beschreibbare und auslesbare Speichereinrichtung derart ausgebildet ist, dass Daten vom Instrument hinsichtlich Sterilisationszyklen, Haltbarkeitsdaten, Instrumentenerkennung oder dergleichen Daten einlesbar und auslesbar sind. Diese Daten lassen sich z. B. unmittelbar über die Schreib- und/oder Leseeinrichtung eingeben oder aber die Messeinrichtung erfasst die Daten und hinterlegt diese in der beschreibbaren und auslesbaren Speichereinrichtung des Instruments. In jedem Falle können die Daten dann bei Bedarf über die Schreib- und/oder Leseeinrichtung zur Verfügung gestellt werden.
Die Messeinrichtung kann z. B. derart ausgebildet sein, dass sie Messwerte z. B. bzgl. eines Sterilisationszyklus erfasst (ggf. von einem Autoklaven, der ebenfalls eine Schreib-und/oder Leseeinrichtung aufweist), so dass diese Daten jederzeit, z. B. über das Chirurgiegerät auslesbar sind.

Vorzugsweise umfasst das Instrument mindestens ein Codierungselement, das der beschreibbaren und auslesbaren Speichereinrichtung zugeordnet ist und das derart ausgebildet ist, dass eine Codierung, vorzugsweise zur Erkennung des Instruments, von der Schreib- und/oder Leseeinrichtung am Chirurgiegerät erfassbar ist. Auch mit dieser Ausführungsform können die entsprechenden Daten unmittelbar ausgelesen werden oder aber die Messeinrichtung erfasst die entsprechende Codierung.

Vorzugsweise ist die Schreib- und/oder Leseeinrichtung mit der Geräteantenne derart ausgebildet, dass die Daten vom Instrument und/oder die Codierung durch eine Verpackung des Instruments hindurch, insbesondere durch eine sterile Verpackung hindurch, erfassbar sind. Das heißt, die Instrumente können durch die Verpackung geprüft werden. So kann eine Überprüfung hinsichtlich der Instrumentenart (Instrumentenerkennung), des Sterilitätszustandes, der Anzahl der Sterilisationszyklen oder dergleichen Parameter erfolgen. Es ist hier also auf eine geeignete Sendeleistung zu achten, so dass die Daten erfassbar sind.

Als erfinderisch wird auch die Verwendung einer beschreibbaren und auslesbaren, mit einer Instrumentenantenne in Verbindung stehenden Speichereinrichtung, insbesondere eines RFID-Transponders, in einem chirurgischen Instrument in verpacktem Zustand, zur Erfassung bzw. zum Einlesen und Auslesen von Instrumentendaten und deren Übermittlung an einen Empfänger betrachtet.

Ist der Empfänger als eine Schreib- und/oder Leseeinrichtung eines Chirurgiegerätes ausgebildet, die mit einer Geräteantenne in Verbindung steht, so können die Daten vom Instrument unmittelbar an das Chirurgiegerät bzw. an die Schreib- und/oder Leseeinrichtung übermittelt werden bzw. von diesem angefordert werden, auch wenn das Instrument noch nicht zur Anwendung mit dem Chirurgiegerät verbunden ist. Die Schreib- und/oder Leseeinrichtung ist z. B. der Steuerungs- und Auswerteeinrichtung des Chirurgiegerätes zugeordnet, die die Daten vom Instrument entsprechend auswertet und ggf. weitere Vorgänge aufgrund der empfangenen Daten auslöst (und sei es nur die Anzeige der Informationen auf einem Display). Vorteilhaft ist es insbesondere, wenn Daten über den Sterilitätszustand des Instruments ermittelt werden, so dass der Anwender erkennt, ob das Instrument einer sachgemäßen Sterilisation unterzogen wurde oder nicht.

Vorrichtungsmäßig wird die Aufgabe weiterhin dadurch gelöst, dass eine Vorrichtung zur kontaktlosen Kommunikation zwischen einem Empfänger, insbesondere einem Chirurgiegerät, und mindestens einem mit diesem anwendbaren chirurgischen Instrument oder dergleichen Zubehöreinrichtung vorgesehen ist, wobei der Empfänger eine Steuerungs-und Auswerteeinrichtung aufweist, der mindestens eine Schreib- und/oder Leseeinrichtung zugeordnet ist, die mit einer Geräteantenne in Verbindung steht, wobei dem Instrument mindestens eine, insbesondere als RFID-Transponder ausgebildete, beschreibbare und auslesbare Speichereinrichtung zugeordnet ist (bzw. der Transponder umfasst die Speichereinrichtung), die mit einer Instrumentenantenne in Verbindung steht, so dass Daten zwischen Empfänger und der Speichereinrichtung des Instruments über eine drahtlose Kommunikation austauschbar sind, wobei die Schreib- und/oder Leseeinrichtung mit der Geräteantenne derart ausgebildet ist, dass die Kommunikation zwischen dem Empfänger und einem verpackten Instrument, insbesondere einem steril verpackten Instrument, erfolgen kann.

Als Empfänger ist hier nicht allein die Schreib- und/oder Leseeinrichtung mit der Antenne gemeint, sondern umfassender, die Vorrichtung, die die Schreib- und/oder Leseeinrichtung mit der Antenne aufweist.

Mit dieser Vorrichtung lassen sich verpackte Instrumente überprüfen, insbesondere hinsichtlich ihres Sterilitätszustandes. Das heißt, es können sterile Produkte mit einem limitierten "Haltbarkeitsdatum" ohne Beschädigung der Sterilverpackung elektronisch auf Verwendbarkeit geprüft oder bei einem Einsatz nach Ablauf der Frist vom Gerät zurückgewiesen werden. Auch wenn sich mehrere Zubehörteile in einer Verpackungseinheit befinden, können diese erkannt und unterschieden werden. Somit ist es möglich, eine Verpackung mit sterilisierten Instrumenten auf Vollständigkeit zu überprüfen, ohne die Sterilverpackung öffnen zu müssen. Dies stellt besonders beim Ablauf im Krankenhaus sowie auch in der gesamten Logistik einen erheblichen Vorteil dar. Hierzu bedarf es noch keiner elektrischen Verbindung des Instruments mit dem Chirurgiegerät. Vielmehr können über die Schreib- und/oder Leseeinrichtung mit der Geräteantenne Informationen über das Instrument ausgelesen werden, um z. B. zu prüfen, ob das Instrument für den angestrebten Einsatz geeignet ist. So können neben einer Instrumentenerkennung auch - wie bereits oben beschrieben - Daten über den Sterilitätszustand entnommen werden, ohne das Instrument aus der Verpackung lösen zu müssen.

Durch die drahtlose Speichertechnik ist es unabhängig vom Einbauort des RFID-Systems im Instrument bzw. im Zubehörteil möglich, die Anzahl der absolvierten Sterilisationszyklen genau zu überwachen. Dazu muss lediglich am Autoklaven eine entsprechende Schreib- und/oder Leseeinrichtung vorgesehen werden, welche die Daten des Tags beim Sterilisieren ausliest, einen abgespeicherten Sterilisations-Zähler verändert und die Werte wieder zurückschreibt. Die Instrumente können also bei der Sterilisation kontaktlos erkannt und neu programmiert werden. Somit kann ein "echter" Sterilisationszyklenzähler implementiert werden, indem im RFID-System ein RFID-Terminal integriert wird, welches auf eine Zählfunktion im Tag zugreift.

In einer Ausführungsform können die Tags u. a. in Folienform oder als sehr preiswerte, druckbare Polymerschaltung auf z. B. einer Neutralelektrode vorgesehen sein. Polymerschaltkreise sind so preisgünstig, dass der Einsatz auf Elektroden für den Einmalgebrauch denkbar ist. Die Leseeinheit befindet sich bei dieser Anordnung bevorzugterweise in der Klemmvorrichtung des Neutralelektrodenkabels, so dass die Elektrode direkt erkannt werden kann. Damit ist es möglich, den genauen Typ der Neutralelektrode zu bestimmen.

Weitere Ausführungsformen der Erfindung ergeben sich aus den Unteransprüchen.

Nachfolgend wird die Erfindung anhand von Ausführungsbeispielen beschrieben, die anhand der Abbildungen näher erläutert werden. Hierbei zeigen:
- - Fig. 1: eine Ausführungsform der erfindungsgemäßen Vorrichtung mit einem Chirurgiegerät und einem daran angeschlossenem Instrument;
- - Fig. 2: eine vereinfachte Darstellung der erfindungsgemäßen Vorrichtung mit Schreib- und/oder Leseeinrichtung und beschreibbarer und auslesbarer Speichereinrichtung zur Erläuterung des Grundprinzips der erfindungsgemäßen Vorrichtung;
- - Fig. 3: eine vereinfachte Darstellung der erfindungsgemäßen Vorrichtung mit Chirurgiegerät und Instrument zur Erläuterung der Messfunktion (i. W.) gemäß Fig. 1;
- - Fig. 4: ein Ablaufdiagramm zur Erläuterung der Messfunktion der erfindungsgemäßen Vorrichtung, wie es mit Fig. 3 dargestellt ist;
- - Fig. 5: ein Ablaufdiagramm entsprechend Fig. 4, in allgemeiner Darstellung;
- - Fig. 6: eine vereinfachte Darstellung der erfindungsgemäßen Vorrichtung in einer weiteren Ausführungsform;
- - Fig. 7: eine weitere vereinfachte Darstellung der erfindungsgemäßen Vorrichtung (i. W.) gemäß Fig. 1;
- - Fig. 8: eine vereinfachte Darstellung der erfindungsgemäßen Vorrichtung in einer weiteren Ausführungsform;
- - Fig. 9: eine vereinfachte Darstellung der erfindungsgemäßen Vorrichtung in einer weiteren Ausführungsform;
- - Fig. 10: eine vereinfachte Darstellung der erfindungsgemäßen Vorrichtung in einer weiteren Ausführungsform;
- - Fig. 11: eine vereinfachte Darstellung der erfindungsgemäßen Vorrichtung in einer weiteren Ausführungsform.

In der nachfolgenden Beschreibung werden für gleiche und gleich wirkende Teile dieselben Bezugsziffern verwendet.

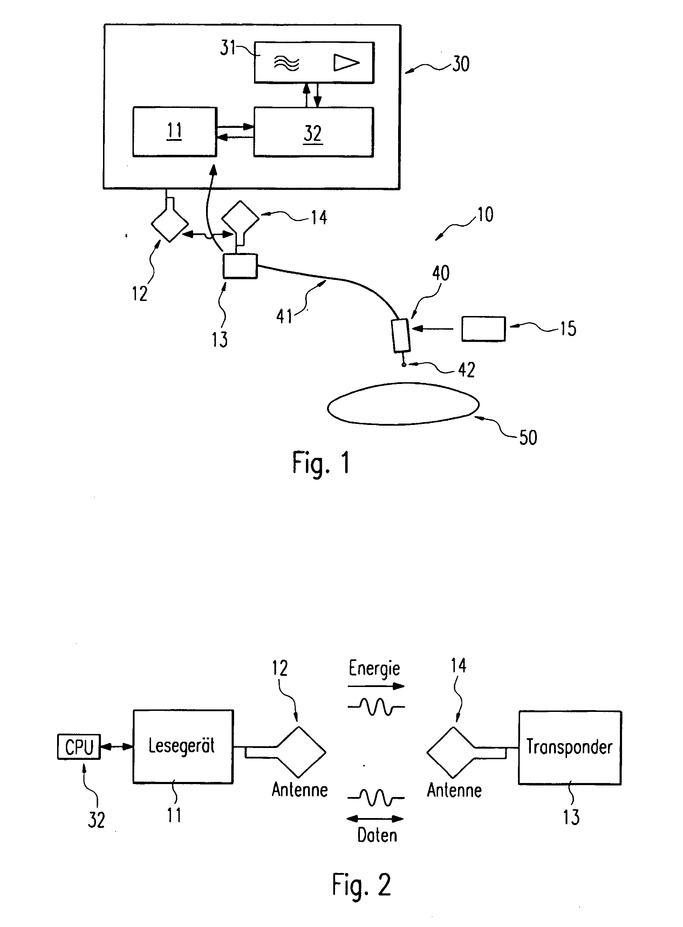

Fig. 1 zeigt eine erste Ausführungsform der erfindungsgemäßen Anordnung mit einem RFID-System. Gezeigt ist ein Chirurgiegerät 30 mit einem daran angeschlossenen chirurgischen Instrument 40 zur Behandlung eines biologischen Gewebes 50. Das Instrument ist hier für die monopolare HF-Chirurgie vorgesehen und weist daher eine Nadelelektrode 42 auf (eine entsprechende Neutralelektrode ist nicht gezeigt). Das Chirurgiegerät 30 umfasst einen HF-Generator 31 zur Bereitstellung hochfrequenten Stroms. In dem Chirurgiegerät 30 ist eine Schreib- und/oder Leseeinrichtung 11 angeordnet, der wiederum eine Geräteantenne 12 zugeordnet ist. Die Schreib- und/oder Leseeinrichtung 11 steht mit einer Steuerungs- und Auswerteeinrichtung 32 in Verbindung, die Steuerungs- und Auswerteeinrichtung 32 steht wiederum mit dem HF-Generator 31 in Verbindung. Schreib- und/oder Leseeinrichtung 11 und Steuerungs- und Auswerteeinrichtung 32 können auch integral miteinander ausgebildet sein.

Das Instrument 40 bzw. ein Instrumentenstecker zum Verbinden des Instruments mit dem Chirurgiegerät 30 ist mit einer beschreibbaren und auslesbaren Speichereinrichtung 13, hier z. B. mit einem RFID-Transponder oder Tag mit einer Instrumentenantenne 14 ausgebildet. Dem Instrument 40 ist eine Mess- und/oder Aktuatoreinrichtung 15 zugeordnet, der wiederum z. B. ein Sensor und/oder ein Aktuator am und/oder im Instrument (beide nicht gezeigt) zugeordnet ist bzw. sind.

Das chirurgische Instrument 40 wird über den HF-Generator 31 mit Strom durch ein Instrumentenkabel 41 versorgt, so dass eine entsprechende Behandlung am Patienten (z. B. Koagulation) vornehmbar ist. Der Mess- und/oder Aktuatoreinrichtung 15 ist hier z. B. ein Sensor zugeordnet, der z. B. die Temperatur an der Elektrode 42 misst. Über diesen Temperaturwert lassen sich Aussagen über den Fortschritt der Behandlung oder über mögliche Gefahren (beispielsweise Verbrennungsgefahr) treffen. Die Verbindung des Instruments mit dem HF-Chirurgiegerät über das Kabel 41 ist hier nur durch den in das Gerät weisenden Pfeil angedeutet.

Da die Instrumente über Standardkabel mit z. B. 3-Pin-Steckern betrieben werden, ist es nun schwierig, zusätzliche Informationen, wie beispielsweise den Messwert (hier Temperatur) ebenfalls über das Kabel zu übertragen. Insofern sind die Schreib- und/oder Leseeinrichtung 11 mit Geräteantenne 12 und die beschreibbare und auslesbare Speichereinrichtung 13 mit Instrumentenantenne 14 vorgesehen, über die die zusätzlichen Informationen per Funk, also per drahtlose Kommunikation durch RFID-Technologie übertragen werden. Befindet sich der Transponder im Stecker des Instrumentenkabels, ist eine optimale Übertragung der Informationen gewährleistet, da Geräteantenne und Instrumentenantenne nah zueinander angeordnet sind. Grundsätzlich ließe sich der Transponder aber auch am Instrument anordnen.

Der vom Instrument 40 zum Chirurgiegerät 30 übermittelte mindestens eine Messwert kann nun über die Steuerungs- und Auswerteeinrichtung 32 ausgewertet und derart "weitergeleitet" werden, dass beispielsweise die Spannung am HF-Generator 31 selbsttätig - aufgrund der erfassten Temperatur - verändert wird, sofern dies notwendig ist.

Die Schreib- und/oder Leseeinrichtung 11 mit der Geräteantenne 12 und die beschreibbare und auslesbare Speichereinrichtung 13 mit der Instrumentenantenne 14 bilden den wesentlichen Teil der Vorrichtung 10 zur kontaktlosen Kommunikation (RFID-System) zwischen Chirurgiegerät 30 und Instrument 40. Dieser Vorrichtung ist dann die Mess-und/oder Aktuatoreinrichtung zugeordnet, über welche zusätzliche Informationen bzw. Daten aufnehmbar und auch auslesbar sind, um ggf. Reaktionen am Chirurgiegerät und/oder am Instrument zu bewirken.

Fig. 2 zeigt eine vereinfachte Darstellung der erfindungsgemäßen Vorrichtung zur Erläuterung des Grundprinzips der drahtlosen Kommunikation. Transponder 13 mit Instrumentenantenne 14 und Schreib- und/oder Leseeinrichtung 11 mit Geräteantenne 12 wirken derart zusammen, dass einerseits Daten in beide Richtungen per Funk übertragbar sind, andererseits aber auch Energie, zumindest vom Chirurgiegerät zum Instrument.

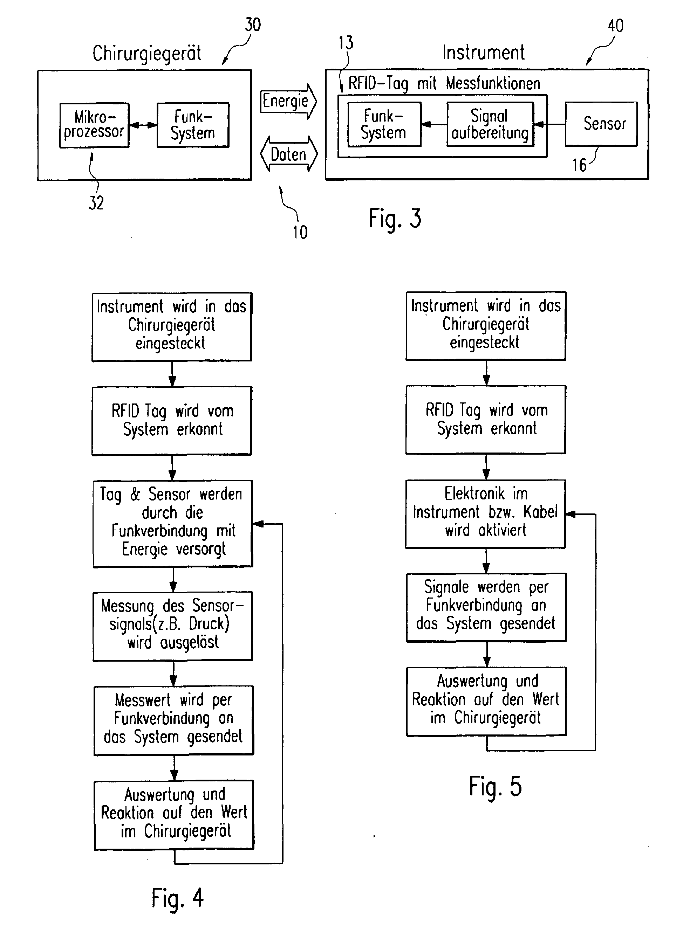

Fig. 3 zeigt eine vereinfachte Darstellung der erfindungsgemäßen Vorrichtung mit Chirurgiegerät 30 und Instrument 40 zur Erläuterung der Messfunktion gemäß Fig. 1. Mit Funksystem bzw. RFID-System ist hier insbesondere die Schreib- und/oder Leseeinrichtung 11 mit Geräteantenne 12 einerseits und die beschreibbare und auslesbare Speichereinrichtung 13 mit Instrumentenantenne 14 andererseits gemeint. Der Sensor 16 nimmt ein Sensorsignal am und/oder im Instrument 40 und/oder am zu behandelnden Gewebe 50 auf, dieses wird entsprechend aufbereitet und über das Funksystem an das Chirurgiegerät 30 übermittelt. Die Steuerungs- und Auswerteeinrichtung 32, z. B. ein Mikroprozessor, stellt das übermittelte Signal derart bereit, dass Eintellungen am Chirurgiegerät 30 vorgenommen werden, die den optimalen Betrieb des Instruments 40 als Rückkopplung auf den mindestens einen erfassten Messwert gewährleisten. Die Einstellung kann dabei manuell erfolgen (aufgrund einer Anzeige der Messung auf einem Display) oder aber die Einstellungen am Chirurgiegerät erfolgen selbsttätig. Hierdurch werden Entscheidungsaufgaben vom Operateur ferngehalten.

Fig. 4 stellt den Ablauf der Funkübertragung in einem Ablaufdiagramm dar, wobei auch die Funkverbindung näher dargestellt ist: Das vom Operateur gewünschte Instrument wird also an das Chirurgiegerät angesteckt. Der RFID-Tag wird vom System erkannt, das heißt, Inhalte der beschreibbaren und auslesbaren Speichereinrichtung werden durch die Schreib- und/oder Leseeinrichtung ausgelesen. Durch die Funkverbindung können Tag und Sensor mit Energie versorgt werden, so dass z. B. eine Messung der Temperatur oder des Drucks (z.B. in der Wasserstrahlchirurgie) über die Messeinrichtung erfolgen kann. Der so ermittelte Wert wird schließlich über Funk wieder an das Chirurgiegerät bzw. an die Schreib- und/oder Leseeinrichtung übermittelt, so dass das Chirurgiegerät (in diesem Falle der HF-Generator) aufgrund der Messung entsprechend geregelt bzw. gesteuert werden kann und das Instrument so z.B. mit den entsprechenden Parametern versorgbar ist.

Das Erkennen des Tags stellt im einfachsten Fall ein Auslesen der Speichereinrichtung dar (um z. B. das Instrument zu erkennen), kann aber auch alle weiteren erforderlichen Vorgänge initiieren, sofern diese vorgesehen sind (also z. B. die Aktivierung der Messeinrichtung).

Fig. 5 zeigt ein Ablaufdiagramm entsprechend Fig. 4, in allgemeiner Darstellung. Diese Darstellung entspricht im Wesentlichen derjenigen gemäß Fig. 4, allerdings ist hier die Messeinrichtung bzw. der Sensor allgemein durch eine "Elektronik" ersetzt. Damit wird deutlich, dass nicht nur eine Messeinrichtung zusätzliche Informationen liefern kann, die per Funkverbindung an das Chirurgiegerät übermittelt werden sollen. Vielmehr soll mit dieser Darstellung gezeigt werden, dass das Transponder-Bauteil nicht nur den beschreibbaren und auslesbaren Speicherbereich aufweist, sondern zusätzliche digitale und analoge Schnittstellen, mit denen elektronische Baugruppen ganz allgemein am und/oder im Instrument betrieben und ausgewertet werden können. Beispielsweise können hier auch Aktoren vorgesehen sein, die Signale vom Chirurgiegerät empfangen und so eine Funktion am Instrument übernehmen.

Die nach oben weisenden Pfeile (sowohl in Fig. 4, als auch in Fig. 5) zeigen auf, dass die Regelung bzw. Steuerung nach einer durchlaufenen Schleife wiederholbar ist.

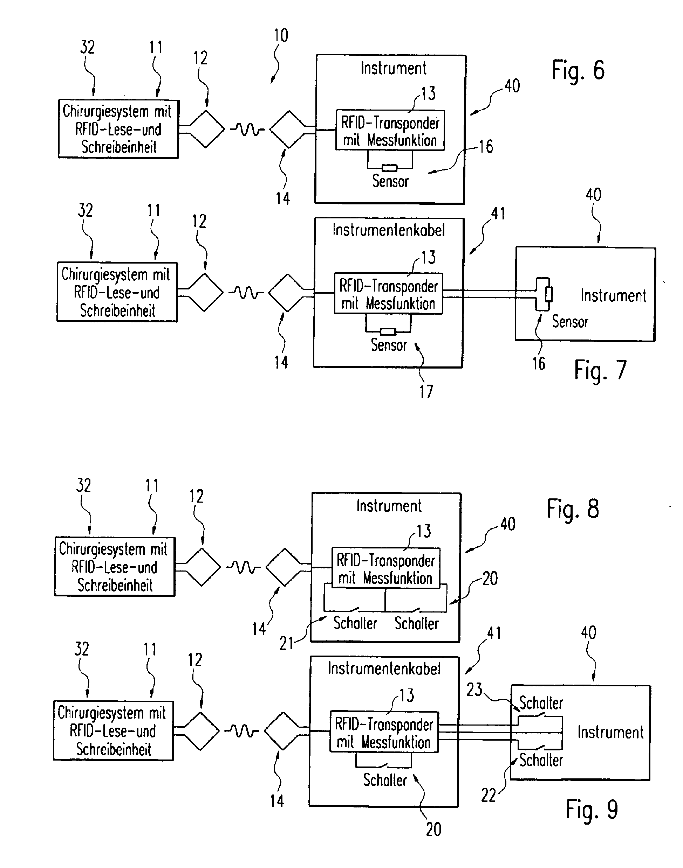

Fig. 6 zeigt eine vereinfachte Darstellung der erfindungsgemäßen Vorrichtung in einer weiteren Ausführungsform. Die Funktionsweise der RFID-Anordnung ist bereits oben beschrieben. Auch hier umfasst das Instrument eine Mess- und/oder Aktuatoreinrichtung 15. Dieser ist z. B. ein im Instrument 40 angeordneter Sensor 16 zugeordnet, der z. B. die Temperatur einer Elektrode oder den Wasserdruck einer Applikationssonde für die Wasserstrahlchirurgie aufnehmen kann. Aufgrund des gemessenen Wertes, der per Funk an die Schreib- und/oder Leseeinrichtung 11 des Chirurgiegerätes 30 übermittelt wird, kann dieses dann nach entsprechender Einstellung die geeigneten Parameter für den weiteren Betrieb des Instruments 40 zur Verfügung stellen. Die Steuerungs- und Auswerteeinrichtung ist hier nicht explizit gezeigt.

Aufgrund der Übermittlung des Messwertes und des darauf folgenden Einstellens des Chirurgiegerätes 30 werden die geeigneten Parameter zum Betrieb des Instruments 40 dann z. B. über das Instrumentenkabel an das Instrument 40 herangeführt. Es ist auch möglich, die Stromversorgung der Elektronik auf der Instrumentenseite in einem bestimmten Rahmen per Funk zur Verfügung zu stellen. Natürlich lassen sich auch Tags einsetzen, deren Stromversorgung z. B. über Batterie erfolgt. Ferner kann lediglich ein Befehl zur Betätigung des Instruments 40 per Funk vom Chirurgiegerät 30 an das Instrument 40 übermittelt werden. Sämtliche Kombinationen der Informationsübertragungen sind hier denkbar.

Fig. 7 zeigt eine weitere vereinfachte Darstellung der erfindungsgemäßen Vorrichtung, wie sie ebenfalls im Wesentlichen in Fig. 1 dargestellt ist. Die Anordnung ist eine ähnliche, wie mit Fig. 6 gezeigt, allerdings ist das Transponder-Bauteil 13 hier im Instrumentenkabel 41, bevorzugt in einem Stecker zum Verbinden des Instruments 40 mit dem Chirurgiegerät 30 ausgebildet. Somit ist eine optimale Funkverbindung gewährleistet, weil die Antennen 12, 14 nah und in im Wesentlichen fixierter Position zueinander angeordnet sind. Der Sensor 16 ist hier im Instrument vorgesehen, um z. B. die Temperatur einer Elektrode während einer Behandlung zu erfassen. Zusätzlich ist hier ein Sensor 17 im Instrumentenkabel angeordnet, um z. B. die Überprüfung der Steckverbindung zu erfassen. Auch dies kann über die Mess- und/oder Aktuatoreinrichtung initiiert werden.

Fig. 8 zeigt eine vereinfachte Darstellung der Vorrichtung in einer weiteren Ausführungsform. Statt Sensoren sind hier z. B. Schalter 20, 21 am chirurgischen Instrument 40 gezeigt. Die Messeinrichtung bzw. die Mess- und/oder Aktuatoreinrichtung 15 kann hier z. B. die Betätigung eines oder der Schalter 20, 21 durch den Operateur feststellen, so dass eine entsprechende Information an die Schreib- und/oder Leseeinrichtung 11 des Chirurgiegerätes 30 übermittelt werden kann. Daraufhin können erforderliche Einstellungen am Chirurgiegerät (in Verbindung mit den betätigten Schaltern) vorgenommen werden - selbsttätig durch das System oder durch den Operateur, z. B. weil entsprechende Daten am Chirurgiegerät angezeigt werden.

Möglich ist es auch, dass der Schalter 20, 21 als Aktuator dient, der über das Chirurgiegerät per RFID-System betätigt werden kann. Grundsätzlich ließe sich der Aktuator natürlich auch über eine herkömmliche Signalleitung ansteuern (weil z. B. ein vom Instrument an das Chirurgiegerät über das RFID-System übermittelter Parameter dies verlangt).

Fig. 9 zeigt eine vereinfachte Darstellung der Vorrichtung in einer weiteren Ausführungsform. Die Anordnung ist ähnlich der in Fig. 8 gezeigten, allerdings ist auch hier das Transponder-Bauteil 13 wieder im Instrumentkabel 41, z. B. im Stecker angeordnet. Ein erster Schalter 20 ist ebenfalls im Stecker angeordnet, während ein zweiter und ein dritter Schalter 22, 23 am Instrument 40 vorgesehen sind. Hier kann die Betätigung eines der Schalter durch den Anwender, z. B. des Schalters 20 am Stecker, die Messeinrichtung 15 aktivieren. Eine Betätigung der Schalter 22, 23 am Instrument 40 (z. B. zu dessen Aktivierung) kann z. B. durch die Messeinrichtung 15 des Instruments 40 erfasst werden, so dass ein entsprechendes Signal über das RFID-System an das Chirurgiegerät (bzw. die Schreib- und/oder Leseeinrichtung) übermittelbar ist.

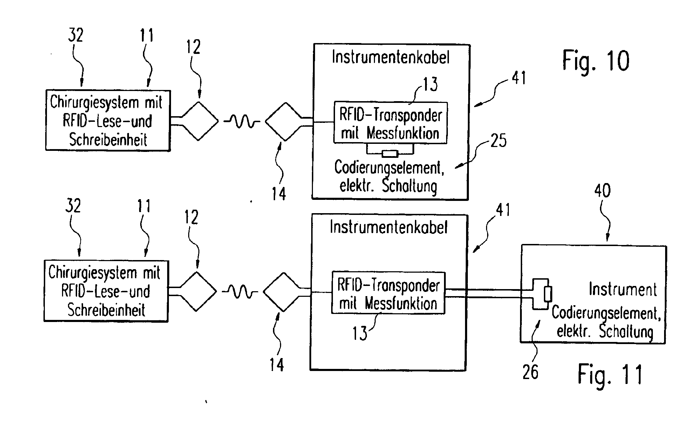

Die Fig. 10 und 11 zeigen weitere Ausführungsformen der Erfindung. Mit Fig. 10 ist gezeigt, dass ein Codierungselement 25 (oder allgemein eine elektronische bzw. elektrische Schaltung) z. B. im Instrumentenkabel 41 zusammen mit der beschreibbaren und auslesbaren Speichereinrichtung 13 angeordnet ist. Das Codierungselement 25 gibt z. B. Auskunft über die Art des Instruments (Instrumentenkennung), so dass mit dem Einstecken des Instruments 40 in das Chirurgiegerät 30 automatisch alle erforderlichen Parameter zum Betreiben des Instruments am Chirurgiegerät eingestellt werden.

In Fig. 11 ist ein Codierungselement 26 im Instrument 40 selbst angeordnet und kann z. B. durch die Messeinrichtung 15 erfasst werden, so dass die entsprechende Information an das Chirurgiegerät 30 übermittelbar sind.

Der Betrieb der Anordnung gemäß der Fig. 10 und 11 ist auch ohne eine Mess- und/oder Aktuatoreinrichtung möglich. Die Anordnung gemäß der Fig. 8 und 9 ist ebenfalls ohne eine explizite Messeinrichtung möglich; so kann also auch nur die Aktuatoreinrichtung vorgesehen sein.

Über das RFID-System ist es ebenfalls möglich, eine Speicherung von Betriebsdaten während der Anwendung des chirurgischen Systems sicherzustellen. So kann z. B. die Einhaltung einer maximal zulässigen Anzahl von Verwendungen des Instruments bzw. eines Zubehörteils überprüft werden. Möglich ist es auch, weitere Informationen in die Speichereinrichtung aufzunehmen, also z. B. Betriebsdaten, die sich während einer Behandlung als zweckmäßig herausstellen. Diese Daten können dann ggf. als Grundlage für weitere Behandlungen dienen oder sie dienen als Vergleich für aktuell aufgenommene Messwerte und bilden so eine Art Referenzbasis.

Mit der erfindungsgemäßen Anordnung ist es möglich, eine Vielzahl an Informationen zwischen einem Instrument und einem Empfänger, insbesondere einer Schreib- und/oder Leseeinrichtung mit entsprechender Antenne in einem Chirurgiegerät, zu übermitteln, ohne dass zusätzliche Verbindungsleitungen erforderlich wären. Da die Datenübermittlung in beide Richtungen möglich ist, können einerseits Informationen vom Instrument an das Chirurgiegerät und andererseits vom Chirurgiegerät an das Instrument per Funk übermittelt werden. Die Versorgung der Messeinrichtung bzw. der Mess- und/oder Aktuatoreinrichtung kann ebenfalls zumindest teilweise per Funk erfolgen.

Der Empfänger muss nicht unbedingt einem Chirurgiegerät zugeordnet sein. Auch ein Autoklav kann eine entsprechende Empfängereinrichtung aufweisen. Damit wäre es möglich, Daten eines Instruments zu erfassen oder in die Speichereinrichtung einzugeben, auch wenn das Instrument nicht mit dem Chirurgiegerät, insbesondere über eine elektrische Verbindung, verbunden ist.

### Bezugszeichenliste

- 10: Vorrichtung zur kontaktlosen Kommunikation
- 11: Schreib- und/oder Leseeinrichtung
- 12: Geräteantenne
- 13: Beschreibbare und auslesbare Speichereinrichtung, Transponder
- 14: Instrumentenantenne
- 15: Mess- und/oder Aktuatoreinrichtung
- 16: Sensor
- 17: Sensor
- 20: Schalter
- 21: Schalter
- 22: Schalter
- 23: Schalter
- 25: Codierungselement
- 26: Codierungselement
- 30: Chirurgiegerät
- 31: HF-Generator
- 32: Steuerungs- und Auswerteeinrichtung, Mikroprozessor, CPU
- 40: Chirurgisches Instrument
- 41: Instrumentenkabel mit Stecker
- 42: Elektrode
- 50: Zu behandelndes Gewebe

## Patentansprüche

1. Vorrichtung zur kontaktlosen Kommunikation zwischen einem Chirurgiegerät (30) und mindestens einem mit diesem anwendbaren chirurgischen Instrument (40), wobei das Chirurgiegerät (30) eine Steuerungs- und Auswerteeinrichtung (32) aufweist,
der mindestens eine Leseeinrichtung (11) zugeordnet ist, die mit einer Geräteantenne (12) in Verbindung steht,
wobei dem Instrument (40) mindestens eine beschreibbare und auslesbare Speichereinrichtung (13) zugeordnet ist, die mit einer Instrumentenantenne (14) in Verbindung steht und mit der Instrumentenantenne (14) als ein Transponder-IC mit einem frei programmierbaren Mikroprozessor ausgebildet ist,
so dass Daten zwischen der Leseeinrichtung (11) des Chirurgiegeräts (30) und der Speichereinrichtung (13) des Instruments (40) über eine drahtlose Kommunikation nämlich über eine Funkverbindung austauschbar sind,
wobei das Instrument (40) eine Messeinrichtung (15) umfasst, die der Speichereinrichtung (13) zugeordnet ist und die derart die Speichereinrichtung (13) beeinflussend ausgebildet ist,
dass mindestens ein Parameter am und/oder im Instrument (40) und/oder an einem zu behandelnden Gewebe (50) zur Übermittlung an das Chirurgiegerät (30) erfassbar ist, wobei der Messeinrichtung (15) mindestens ein instrumentenseitig angeordneter Sensor (16) zugeordnet ist und die Messeinrichtung (15) derart ausgebildet ist, dass sie ein Sensorsignal mindestens eines Sensors (16) als den Parameter erfasst, wobei durch die Funkverbindung der Transponder-IC und der Sensor mit Energie versorgt werden.

2. Vorrichtung nach Anspruch 1,
**dadurch gekennzeichnet, dass**
die beschreibbare und auslesbare Speichereinrichtung (13) mit der Instrumentenantenne (14) in einer Steckereinrichtung oder in einem Verbindungskabel (41) zum Verbinden des Instruments (40) mit dem Chirurgiegerät (30) ausgebildet ist.

3. Vorrichtung nach einem der vorhergehenden Ansprüche,
**dadurch gekennzeichnet, dass**
die Mess einrichtung (15) im Instrument (40) oder in dem Verbindungskabel (41) zum Verbinden des Instruments (40) mit dem Chirurgiegerät (30) ausgebildet ist.

4. Vorrichtung nach einem der vorhergehenden Ansprüche,
**dadurch gekennzeichnet, dass**
das Chirurgiegerät (30) und das Instrument (40) für die Wasserstrahlchirurgie ausgebildet sind und die Messeinrichtung (15) derart ausgebildet und angeordnet ist, dass der Druck in einer Zuleitung für Wasser an das zu behandelnde Gewebe eines Wasserstrahlapplikators als Parameter über den Sensor (16) erfassbar ist.

5. Vorrichtung nach einem der Ansprüche 1 bis 3,
**dadurch gekennzeichnet, dass**
das Chirurgiegerät (30) und das Instrument (40) für die HF-Chirurgie ausgebildet sind und die Messeinrichtung (15) derart ausgebildet und angeordnet ist, dass die Temperatur einer Elektrode (42) eines HF-chirurgischen Instruments als Parameter während einer Benutzungshandlung über den Sensor (16) erfassbar ist.

6. Vorrichtung nach einem der vorhergehenden Ansprüche,
**dadurch gekennzeichnet, dass**
die beschreibbare und auslesbare Speichereinrichtung (13) derart ausgebildet ist, dass Daten vom Instrument (40) hinsichtlich Sterilisationszyklen, Haltbarkeitsdaten, Instrumentenerkennung oder dergleichen Daten einlesbar und auslesbar sind.

## Claims

1. Device for contactless communication between a surgical device (30) and at least one surgical instrument (40) which can be used with the latter, the surgical device (30) having a control and evaluation device (32)
which is assigned at least one reading device (11) which is connected to a device antenna (12),
the instrument (40) being assigned at least one writable and readable storage device (13) which is connected to an instrument antenna (14) and, with the instrument antenna (14), is in the form of a transponder IC with a freely programmable microprocessor,
with the result that data can be interchanged between the reading device (11) of the surgical device (30) and the storage device (13) of the instrument (40) via wireless communication, namely via a radio link,
the instrument (40) comprising a measuring device (15) which is assigned to the storage device (13) and influences the storage device (13) in such a manner
that at least one parameter can be recorded on and/or in the instrument (40) and/or on a tissue (50) to be treated for transmission to the surgical device (30), the measuring device (15) being assigned at least one sensor (16) which is arranged on the instrument side, and the measuring device (15) being designed in such a manner that it records a sensor signal from at least one sensor (16) as the parameter, the transponder IC and the sensor being supplied with energy by the radio link.

2. Device according to Claim 1,
**characterized in that**
the writable and readable storage device (13) is formed, with the instrument antenna (14), in a connector device or in a connecting cable (41) for connecting the instrument (40) to the surgical device (30).

3. Device according to one of the preceding claims,
**characterized in that**
the measuring device (15) is formed in the instrument (40) or in the connecting cable (41) for connecting the instrument (40) to the surgical device (30).

4. Device according to one of the preceding claims,
**characterized in that**
the surgical device (30) and the instrument (40) are designed for water jet surgery, and the measuring device (15) is designed and arranged in such a manner that the pressure in a supply line for water to the tissue to be treated of a water jet applicator can be recorded as the parameter via the sensor (16).

5. Device according to one of Claims 1 to 3,
**characterized in that**
the surgical device (30) and the instrument (40) are designed for HF surgery, and the measuring device (15) is designed and arranged in such a manner that the temperature of an electrode (42) of a HF surgical instrument can be recorded as the
parameter via the sensor (16) during an act of use.

6. Device according to one of the preceding claims,
**characterized in that**
the writable and readable storage device (13) is designed in such a manner that data with respect to sterilization cycles, shelf-life data, instrument detection or such data can be read in and out by the instrument (40).

## Revendications

1. Dispositif de communication sans contact entre un appareil chirurgical (30) et au moins un instrument chirurgical (40) utilisable avec celui-ci, l'appareil chirurgical (30) présentant un dispositif de commande et d'interprétation (32),
auquel est associé au moins un dispositif de lecture (11) qui est en liaison avec une antenne d'appareil (12),
au moins un dispositif à mémoire (13) pouvant être écrit et pouvant être lu étant associé à l'instrument (40), lequel est en liaison avec une antenne d'instrument (14) et, avec l'antenne d'instrument (14), est configuré sous la forme d'un CI transpondeur doté d'un microprocesseur programmable librement,
de sorte que des données peuvent être échangées entre le dispositif de lecture (11) de l'appareil chirurgical (30) et le dispositif à mémoire (13) de l'instrument (40) par le biais d'une communication sans fil, à savoir par le biais d'une liaison hertzienne, l'instrument (40) comprenant un dispositif de mesure (15) qui est associé au dispositif à mémoire (13) et qui est configuré pour influencer le dispositif à mémoire (13) de telle sorte
qu'au moins un paramètre sur et/ou dans l'instrument (40) et/ou sur un tissu (50) à traiter peut être collecté afin d'être communiqué à l'appareil chirurgical (30), au moins un capteur (16) disposé du côté de l'instrument étant associé au dispositif de mesure (15) et le dispositif de mesure (15) étant configuré de telle sorte qu'il collecte un signal de capteur d'au moins un capteur (16) en tant que paramètre, le CI transpondeur et le capteur étant alimentés en énergie par le biais de la liaison hertzienne.

2. Dispositif selon la revendication 1, **caractérisé en ce que** le dispositif à mémoire (13) pouvant être écrit et pouvant être lu muni de l'antenne d'instrument (14) est réalisé dans un dispositif enfichable ou dans un câble de liaison (41) destiné à relier l'instrument (40) avec l'appareil chirurgical (30).

3. Dispositif selon l'une des revendications précédentes, **caractérisé en ce que** le dispositif de mesure (15) est réalisé dans l'instrument (40) ou dans le câble de liaison (41) destiné à relier l'instrument (40) avec l'appareil chirurgical (30).

4. Dispositif selon l'une des revendications précédentes, **caractérisé en ce que** l'appareil chirurgical (30) et l'instrument (40) sont configurés pour la chirurgie par jet d'eau et le dispositif de mesure (15) est configuré et disposé de telle sorte que la pression dans une conduite d'arrivée d'eau vers le tissu à traiter d'un applicateur de jet d'eau peut être collectée en tant que paramètre par le biais du capteur (16).

5. Dispositif selon l'une des revendications 1 à 3, **caractérisé en ce que** l'appareil chirurgical (30) et l'instrument (40) sont configurés pour la chirurgie HF et le dispositif de mesure (15) est configuré et disposé de telle sorte que la température d'une électrode (42) d'un instrument chirurgical HF peut être collectée en tant que paramètre par le biais du capteur (16) pendant une manipulation d'utilisation.

6. Dispositif selon l'une des revendications précédentes, **caractérisé en ce que** le dispositif à mémoire (13) pouvant être écrit et pouvant être lu est configuré de telle sorte que les données de l'instrument (40) concernant les cycles de stérilisation, les données de durabilité, l'identification de l'instrument ou des données similaires peuvent être écrites et être lues.
